# EUROPÄISCHE PATENTSCHRIFT

(11) **EP 0 618 192 B1**
(45) Veröffentlichungstag und Bekanntmachung des Hinweises auf die Patenterteilung: **16.09.1998**
(21) Anmeldenummer: 94104676.5
(22) Anmeldetag: 24.03.1994
(51) Int. Cl.: C07D 207/452, G01N 33/53

(54) **Homobidentale, trifunktionelle Maleinimid-Linker, und ihre Verwendung in immunologisch aktiven Konjugaten**
Homobidental, trifunctional maleimide linkers and their application in immunologically active conjugates
Linkers maléimides homobidentals trifonctionnels et leur application dans des conjugats immunologiquement actifs

(30) Priorität: 29.03.1993 DE 4310141
(43) Veröffentlichungstag der Anmeldung: 05.10.1994
(73) Patentinhaber: BOEHRINGER MANNHEIM GMBH, 68298 Mannheim (DE)
(72) Erfinder: Hoess, Eva, Dr., D-82319 Starnberg (DE); Huber, Erasmus, Dr., D-86923 Finning (DE); Markert-Hahn, Christine Dr., D-82402 Seeshaupt (DE); Ofenloch-Haehnle, Beatus, Dr., D-82407 Wielenbach (DE)

(56) Entgegenhaltungen:
- EP-A- 0 247 866
- EP-A- 0 310 361
- EP-A- 0 446 071
- WO-A-92/22583
- DATABASE WPI Section Ch, Week 8537, Derwent Publications Ltd., London, GB; Class B04, AN 85-226374 & JP-A-60 146 154 (EISAI KK) 1. August 1985

## Beschreibung

Die Erfindung betrifft homobidentale, trifunktionelle Linker, ihre Herstellung und Verwendung zur Herstellung von immunologisch aktiven Substanzen (z. B. Immunogenen, markierten Antikörperkonjugaten) und zur Kopplung von immunologisch aktiven Substanzen aneinander oder an Trägersubstanzen, wie beispielsweise an Proteine oder andere Polymere.

Die Kopplung von immunologisch aktiven Substanzen an Trägerproteine bzw. an in einem Immunoassay nachweisbare Markierungen, erfolgt mit bifunktionellen Linkern oder, wenn mehr als zwei Moleküle verknüpft werden sollen oder zur Bindung eines Moleküls zwei Bindungsstellen erforderlich sind, auch mit trifunktionellen Linkern. So wird beispielsweise im US-Patent Nr. 5,082,930 ein trifunktioneller Linker beschrieben, mit dem Antikörper an radioaktive Metallionen gekoppelt werden können. Dabei wird ein Antikörper kovalent an eine Funktion des Linkers gebunden, während ein radioaktives Metallion über die beiden anderen Funktionen des Linkers in Form eines Chelatkomplexes gebunden wird.

Aus der EP-A 0 310 361 sind trifunktionelle Konjugate bekannt, in denen mindestens zwei der Linkerfunktionen an kleine Moleküle gebunden sind. Länge und chemischer Aufbau der drei Linkerarme sind so beschaffen, daß eine immunologische Bindung von mehreren Antikörpern an diese kleinen Moleküle sterisch behindert werden. Auf Basis dieser Behinderung kann ein Immunoassay durchgeführt werden.

Aus der EP-A 0 446 071 sind ebenfalls trifunktionelle Linker bekannt, deren drei Kopplungsfunktionen identisch sind und jeweils die Maleinimidogruppe darstellen. Mit diesen homotrifunktionellen Linkern können beispielsweise drei Antikörper oder Antikörperfragmente oder auch zwei Antikörper miteinander verbunden werden, wobei in letzterem Fall einer der Antikörper über zwei seiner Thiolgruppen an den trifunktionellen Linker gebunden wird.

Bei der Umsetzung von Proteinen mit solchen homotrifunktionellen Linkern erfolgen jedoch unerwünschte, nicht kontrollierbare Vernetzungen, da die Kupplung der einzelnen Reaktionspartner miteinander in einem Reaktionsschritt durchgeführt werden muß.

Aufgabe der vorliegenden Erfindung war es daher, homobidentale, trifunktionelle Linker zur Verfügung zu stellen, mit denen reproduzierbar und in hoher Ausbeute thiolgruppenhaltige Verbindungen mit Proteinen, Polymeren und Haptenen gekoppelt werden können. Diese Aufgabe wird gelöst durch eine Verbindung der allgemeinen Formel 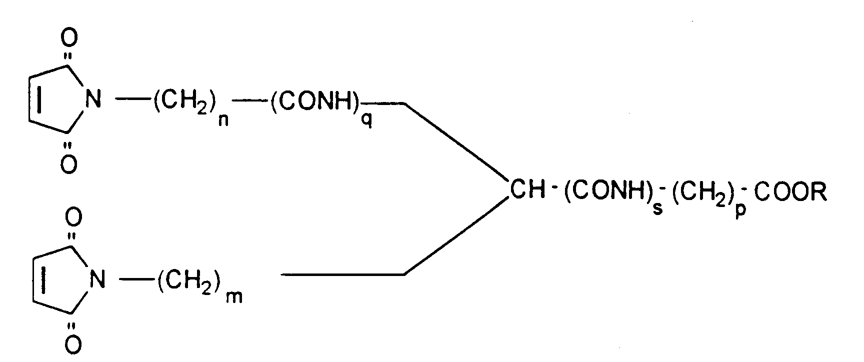 in der R eine esteraktivierende Gruppe, m und n gleich oder verschieden und 0 - 6, q und s 0 oder 1, p = 0, wenn s = 0 ist und p = 2 - 4, wenn s = 1 ist, darstellen.

Die erfindungsgemäßen Verbindungen sind neu. Ihre Herstellung ist nicht durch Analogieverfahren durchführbar.

Es hat sich gezeigt, daß mit derartigen Verbindungen eine stabile Kopplung zwischen Haptenen, Proteinen und/oder Polymeren, wie beispielsweise Dextranen, ermöglicht wird und die mit den erfindungsgemäßen Verbindungen hergestellten Konjugate in hoher Ausbeute erhältlich sind. Die Kopplungsausbeute kann nach dem Fachmann geläufigen Methoden (z. B. über die Bestimmung der Anzahl SH-Gruppen im Konjugat nach G.L. Ellman, Arch. Biochem. Biophys. 74 (1958) 443 - 450 oder P.W. Riddles, Anal. Biochem. 94 (1979) 75 - 81 bestimmt werden).

Als esteraktivierende Gruppe R werden bevorzugt N-Hydroxyestergruppen (wie z. B. die Hydroxysuccinimidylgruppe), Imidazolide, Pyridazolide, Aminoalkylcarbonsäuren oder aktivierte Arylestergruppen (z. B. p-Nitrophenylester) verwendet. n, m, p, q und s können im Rahmen der Erfindung alle genannten Werte annehmen. Es hat sich jedoch gezeigt, daß zur Kopplung großer Haptene insbesondere langkettige Linker geeignet sind.

Besonders bevorzugt sind Verbindungen, in denen
a) R: Hydroxysuccinimidyl,
   n = 5, m = 4, p = 2 und q und s = 1 bedeuten und Verbindungen,
b) R: Hydroxysuccinimidyl,
   n und m = 5, p = 0 und q und s = 0 bedeuten.
c) R: Hydroxysuccinimidyl
   n und m = 3, p = 0, q und s = 0
d) R: Hydroxysuccinimidyl
   n = 5, m = 4, p = 0, q = 1, s = 0

Bevorzugt werden die erfindungsgemäßen Linker zur Kopplung von immunologisch aktiven Substanzen, wie Antikörper und Antikörperfragmente, mit Haptenen, Proteinen und Dextran verwendet.

Derartige Kopplungsprodukte können vorteilhaft als Immunogene verwendet werden, da dabei die Kopplung einer doppelten Anzahl von Haptenen an ein Trägerprotein möglich ist. Besonders gute Immunantworten werden nämlich erhalten, wenn das verwendete Immunogen hinreichend hoch mit Hapten beladen ist.

Es ist zudem vorteilhaft, wenn das Immunogen einen basischen pI-Wert hat (A. Muckerheide et al., J. Immunol. 138 (1987) 833; R.J. Apple et al, J. Immunol. 140 (1987) 3290; P.L. Domen, J. Immunol. 139 (1987) 3195). Eine Möglichkeit, den isoelektrischen Punkt zu erhöhen, ist die Einführung von zusätzlichen Aminogruppen in Trägerproteine (EP 0 462 669; A. Muckerheide et al., J. Immunol. 138 (1987), 2800; Pierce Supercarrier 77150). In einem zweiten Schritt erfolgt dann erst die Aktivierung mit Maleinimid- bzw. Mercaptogruppen.

Mit den erfindungsgemäßen Linkern ist es jedoch möglich, eine hinreichend hohe Immunogenbeladung zu erhalten, ohne jedoch alle Aminogruppen des Proteins zu derivatisieren. Über die bei der Reaktion verfügbaren Mengen an Protein kann der Einbau an erfindungsgemäßen Linkern gesteuert werden.

In Immunoassays wird durch den Hapten-Beladungsgrad der Konjugate die Sensitivität entscheidend mitbeeinflußt, so auch bei der Detektion von hochmolekularen Analyten im CEDIA-Prinzip (EP-A 0 497 613). Die Detektion niedrigkonzentrierter Analyten kann nur mit hochbeladenen Antikörper-ED-Konjugaten (ED = Enzymdonor der β-Galactosidase) erfolgen. Die Beladung ist aber durch die Zahl der zugänglichen ε-Aminogruppen des Antikörpers oder Antikörperfragments beschränkt. Durch die erfindungsgemäßen homobidentalen, trifunktionellen Linker kann ohne Vernetzung der Antikörper der Einbau verdoppelt und so die Sensitivität gesteigert werden.

So ist es bevorzugt, zwei ED-Untereinheiten von β-Galactosidase oder zwei Haptene über die beiden Maleinimidogruppen der erfindungsgemäßen Linker an einen Antikörper oder ein Antikörperfragment oder ein Dextran, welcher oder welches über die aktivierte Gruppe R an den erfindungsgemäßen Linker gebunden ist, zu koppeln. In weiteren bevorzugten Konjugaten sind zwei Haptene über die beiden Maleinimidogruppen der erfindungsgemäßen Linker an eine ED-Untereinheit, die über die aktivierte Gruppe R an den erfindungsgemäßen Linker gebunden ist, gekoppelt.

Verfahren zur Aktivierung und Kopplung von Haptenen, Proteinen sowie Antikörpern sind dem Fachmann geläufig. Die Herstellung von Maleinimidderivaten und ihre Kopplung mit SH-Gruppen ist beispielsweise bei O. Keller, Helvetica Chimica Acta 58 (1975) 531 - 541 beschrieben. Säureamide können beispielsweise nach der EP-A 0 029 909 hergestellt werden. Die Aminierung von Proteinen kann beispielsweise nach der EP-A 0 462 669 erfolgen.

Die Synthese von erfindungsgemäßen Linkern, in denen q = O und s = O sind, kann durchgeführt werden durch zweifache Malonester-Synthese mit N-Phtalimidoalkylhalogeniden (vorzugsweise Bromid) an Malondiester (vorzugsweise Malondi-t-butylester). Es wird dabei ein Dialkylmalondiester erhalten. Nach Spaltung des Malondiesters in sauren (vorzugsweise mit Trifluoressigsäure) tritt beim Erwärmen Decarboxylierung (Säurespaltung) auf. Anschließend können durch Behandlung mit Hydrazin oder durch saure Hydrolyse die Aminogruppen freigesetzt werden. Durch Behandlung mit Alkoxycarbonylmaleinimid (bevorzugt Methoxy-oder Ethoxycarbonylmaleinimid (O. Keller, loc. cit.)) werden die beiden Maleinimidylreste eingeführt. Im weiteren wird die Carboxylfunktion nach dem Fachmann bekannten Methoden, beispielsweise mit Dicyclohexylcarbodiimid (DCCI) und N-Hydroxysuccinimid, aktiviert. Durch zusätzliche Umsetzung des aktivierten Esters mit ω-Aminocarbonsäuren (z. B. β-Alanin, U-Aminocapronsäure) und anschließender Aktivierung, beispielsweise mit DCCI und N-Hydroxysuccinimid, können erfindungsgemäße Linker mit q = O und s = 1 erhalten werden. Es wird dadurch ein erfindungsgemäßer Linker erhalten, der zwei Maleinimidylreste und eine Hydroxysuccinimidesterfunktion als R enthält. Andere Reste R können eingeführt werden, indem die Carboxylfunktion nach dem Fachmann bekannten Methoden derivatisiert wird.

Die Synthese von erfindungsgemäßen Linkern mit q und s = 1 kann durchgeführt werden durch Umsetzung einer Diaminosäure, wobei eine der Aminofunktionen durch eine säurelabile Schutzgruppe (vorzugsweise t-Butyloxycarbonyl) geschützt ist, mit Alkoxycarbonylmaleinimiden, bevorzugt mit Methoxy- oder Ethoxycarbonylmaleinimid (O. Keller, loc. sit)). Anschließend wird die erhaltene Verbindung an der freien Carboxyfunktion mit einer Aminosäure umgesetzt.

Handelt es sich um eine α-Diaminocarbonsäure, wird das Molekül zweckmäßig entweder mit einer ω-Aminocarbonsäure (vorzugsweise β-Alanin) oder bereits einer entsprechend Amino-derivatisierten zu koppelnden Verbindung wie einem Hapten (vorzugsweise einem Biotin-Derivat) verknüpft. Die Kupplung der Aminogruppe an die freie Carboxygruppe erfolgt nach dem Fachmann bekannten Kupplungsmethoden (bevorzugt mit Carbodiimiden und N-Hydroxysuccinimid).

Nach Abspaltung der Schutzgruppe unter sauren Bedingungen (z. B. mit Trifluoressigsäure oder 2 N Chlorwasserstoff in Dioxan) wird die erhaltene Verbindung mit einer Carboxyaktivierten Maleinimidoalkansäure (vorzugsweise Maleinimidohexanoxyl-N-hydroxysuccinimid) umgesetzt. Die Aktivierung der freien Carbonylfunktion erfolgt nach dem Fachmann bekannten Methoden, beispielsweise mit Morpholinoethylisocyanid und N-Hydroxysuccinimid.

Die Synthese von erfindungsgemäßen Linkern mit q = 1 und s = 0 kann analog ausgehend von Diaminosäuren durchgeführt werden, wobei im ersten Schritt keine der Aminogruppen durch eine Schutzgruppe geschützt wird.

Die Aktivierung von Proteinen mit trifunktionellen Linkern kann durch nucleophile Substitution der N-Hydroxysuccinimid-Funktion des Linkers durch die ε-Aminogruppe der Lysinseitenketten von Proteinen in leicht alkalischem Puffer erfolgen. Hierzu wird ein 1 - 8facher Überschuß an Linker, bezogen auf die zu derivatisierenden Aminogruppen des Proteins, verwendet. Durch Dialyse oder Gelchromatographie wird das bei der Reaktion gebildete N-Hydroxysuccinimid und der überschüssige Linker abgetrennt.

Aminogruppenhaltige Haptene können entweder in organischen Medien, wie Dioxan oder DMF unter Zusatz von Triethylamin oder in Puffergemischen, bevorzugt Kaliumphosphatpuffer, pH 7,5, mit äquimolaren Mengen erfindungsgemäßem Linker umgesetzt werden.

Die Bestimmung des Proteingehalts in Lösung (zur Ermittlung der Ausbeute der Umsetzung) kann beispielsweise mit dem "BCA protein assay test von Pierce Chemicals (Bestell-Nr. 23225) erfolgen. (P.K. Smith, Anal. Biochem. 150 (1985) 76 - 85)

Die Bestimmung des Beladungsgrades von Maleinimido funktionalisierten Proteinen erfolgt vorzugsweise mit "Ellmans-Reagenz" (5,5'-Dithio-bis-(2-nitrobenzoesäure)). Zur Konjugatsynthese werden beispielsweise SH-aktivierte Haptene und Maleinimido-aktivierte Proteine in der Weise umgesetzt, daß das SH-aktivierte Hapten unter Schutzgasatmosphäre (z. B. Argon) in Kaliumphosphatpuffer im leicht Sauren mit einem Maleinimido-funktionalisierten Protein zur Reaktion gebracht wird. Das Hapten wird dabei unter Ausbildung einer Thioetherbindung an das Protein gebunden. Die Menge des einzusetzenden SH-aktiven Haptens richtet sich nach der Zahl der Maleinimidogruppen des verwendeten Konjugats aus Protein und Linker. Um vollständigen Umsatz zu erzielen, hat sich ein Beladungsangebot von 2:1 als Verhältnis von SH:Maleinimidogruppen bewährt.

Die Erfindung wird durch die folgenden Beispiele näher erläutert:

### Beispiel 1

### 1.1 N-α-Boc-ε-maleinimido-α-aminohexansäure (α-Boc-ε-Mal-Lys)

3.70 g (15 mmol) α-t-Butyloxycarbonyl-Lysin werden in 200 ml ges. Natrium-hydrogen-carbonat-Lösung aufgenommen, mit 2.54 g (15 mmol) N-Ethoxycarbonyl-maleinimid versetzt und 30 min bei 20°C gerührt. Anschließend wird die Reaktionslösung mit 200 ml Wasser verdünnt, mit 2 N Salzsäure auf pH 1.8 gestellt, zweimal mit je 200 ml Essigester extrahiert, über Magnesiumsulfat getrocknet und das Lösungsmittel im Wasserstrahlvakuum abdestilliert. Das Rohprodukt wird mittels offener Säulenchromatographie (5 x 50 cm) über Kieselgel, Eluent Essigester/Methanol (v/v 4/1)/1 % Essigsäure, aufgereinigt. Die Fraktionen, die Produkt enthalten, werden vereinigt, das Lösungsmittel im Wasserstrahlvakuum entfernt und der Rückstand im Hochvakuum über CaCl₂ getrocknet.
- Ausbeute:: 3.8 g (11.58 mmol) 78 % d.Th.
- DC:: Kieselgel (Merck 60), Essigester/Methanol (v/v 3/2)/1% Essigsäure, Detektion mit Kaliumpermanganat R_{f} = 0.83
- ¹H-NMR (D₆-DMSO/TMS):: δ = 1.35 (m, 15H, ^{t}Bu u. 3 CH₂); 3.35 (m, 2H, CH₂-N); 3.66 (m, 1H, CH-N); 6.18 (d, br, 1H, NH, J=7.2 Hz); 6.98 ppm (s, 2H, CH=).

### 1.2 N-α-Boc-ε-maleinimido-α-aminohexanoyl-β-alanin (α-Boc-ε-Mal-Lys-β-Ala)

1.60 g (4.9 mmol) der Verbindung aus Beispiel 1.1 in 200 ml THF werden mit 676 mg (5.92 mmol) N-Hydroxysuccinimid und 1.21 g (5.92 mmol) Dicyclohexylcarbodiimid versetzt und 1 h bei 20°C gerührt. 480 mg (5.4 mmol) β-Alanin werden in 200 ml 0.1 M KPO₄-Puffer pH 8.5 gelöst und zum Reaktionsansatz getropft. Nach weiteren 16 h Rühren bei 20°C wird das organische Lösungsmittel im Wasserstrahlvakuum abdestilliert, die wässrige Phase mit 100 ml Wasser verdünnt, zweimal mit je 250 ml Essigester extrahiert, über Magnesiumsulfat getrocknet und im Wasserstrahlvakuum eingeengt. Der Rückstand wird über eine Kieselgelsäule (5 x 30 cm), Eluent Essigester/Methanol (v/v 4/1)/1 % Essigsäure aufgereinigt.
- Ausbeute:: 1.17 g (2.96 mmol) 61 % d.Th.
- DC:: Kieselgel (Merck 60), Essigester/Methanol (v/v 4/1)/1 % Eisessig, Detektion mit Kaliumpermanganat R_{f} = 0.7
- ¹H-NMR (D₆-DMSO/TMS):: δ = 1.35 (m, 15 H, ^{t}Bu u. 3 CH₂); 2.32 (t, 2H, CH₂CO, J=6.9 Hz); 3.16-3.41 (m, 4H, CH-N); 3.88 (m, 1H, CH-N); 6.73 (d, 1H, NH-COO, J=7.7 Hz); 6.98 (s, 2H, CH=); 7.78 ppm (t, 1H, NH-CO, J=5.4 Hz).

### 1.3 ε-Maleinimido-α-aminohexanoyl-β-alanin (ε-Mal-Lys-β-Ala)

710 mg (1.8 mmol) der Verbindung aus Beispiel 1.2 werden bei 0°C unter Rühren mit 15 ml Trifluoressigsäure versetzt, langsam auf 20°C erwärmt. Nach 30 min wird mit 15 ml Essigester verdünnt, weitere 15 min bei 20°C gerührt, das Lösungsmittel im Wasserstrahlvakuum abdestilliert und der Rückstand aus Dioxan/Wasser (v/v 1/1) lyophilisiert.
- Ausbeute:: 430 mg (1.5 mmol) 81 % d.Th.
- DC:: Kieselgel (Merck 60), Essigester/Methanol (v/v 1/4)/1 % Eisessig, Detektion mit Ninhydrin R_{f} = 0.33
- ¹H-NMR (D₆-DMSO/TMS):: δ = 1.15-1.64 (m, 6H, 3 CH₂); 2.41 (t, 2H, CH₂-CO, J=6.6 Hz); 3.37 (m, 4H, 2 CH₂-N); 3.68 (m, 1H, CH-N); 7.00 (s, 2H, CH=); 8.53 ppm (t, 1H, NH-CO, J=6.0 Hz).

### 1.4 N-α-(6-Maleinimidohexanoyl)-ε-maleinimido-α-aminohexanoyl-β-alanin (α-MHS-e-Mal-Lys-β-Ala)

Zu einer Lösung aus 400 mg (1.35 mmol) der Verbindung aus Beispiel 1.3 in 10 ml 0.1 M KPO₄-Puffer pH 7.5 wird unter Rühren eine Lösung aus 462 mg (1.5 mmol) Maleinimidohexansäure-N-hydroxysuccinimidester (MHS) in 10 ml THF getropft. Der pH-Wert wird bis zur Konstanz mit 1 N Natronlauge auf pH 7.5 nachgestellt. Nach 16 stündigem Rühren bei 20°C wird das organische Lösungsmittel im Wasserstrahlvakuum abdestilliert, der Rückstand mit 10 ml Wasser verdünnt und mit 1 N Salzsäure auf pH 3.0 gestellt und dreimal mit je 30 ml Essigester extrahiert - das Produkt geht in die organische Phase über. Diese wird mit Magnesiumsulfat getrocknet und im Wasserstrahlvakuum eingeengt. Der Rückstand wird mittels offener Säulenchromatographie über Kieselgel, Eluent Essigester/Methanol(v/v 2/1)/1 % Essigsäure, gereinigt, die gepoolten Fraktionen eingedampft und aus 10 ml Essigester/THF (v/v 1/1) mit Diisopropylether ausgefällt. Der Niederschlag wird abfiltriert und im Hochvakuum über CaCl₂ getrocknet.
- Ausbeute:: 230 mg (0.47 mmol) 35 % d.Th.
- DC:: Kieselgel (Merck 60), Essigester/Methanol (v/v 2/1)/1 % Eisessig, Detektion mit Kaliumpermanganat R_{f} = 0.63
- ¹H-NMR (D₆-DMSO/TMS):: δ = 1.14-1.50 (m, 12H, 6 CH₂); 2.09 (t, 2H, CH₂-CO, J=6.6 Hz); 2.22 (t, 2H, CH₂-CO, J=7.5 Hz); 3.37 (m, 6H, CH₂-N); 4.07 (m, 1H, CH-N); 7.00 (s, 2H, CH=); 7.85 ppm (m, 2H, NH-CO).

### 1.5 N-α-(6-Maleinimidohexanoyl)-ε-maleinimido-α-aminohexanoyl-β-alanyl-(N-hydroxysuccinimid) (α-MHS-ε-Mal-Lys-β-Ala-OSu)

180 mg (0.36 mmol) der Verbindung aus Beispiel 1.4 werden in 10 ml absolutem DMF gelöst, mit 49 mg (0.43 mmol) N-Hydroxysuccinimid und 60 µl (0.43 mmol) Morpholinoethylisocyanid (MEI) versetzt und 16 h bei 20°C gerührt, wobei nach jeweils 8 h noch zweimal 49 mg (0.43 mmol) N-Hydroxysuccinimid und 60 µl (0.43 mmol) MEI nachdosiert werden. Zur vollständigen Umsetzung wird noch weitere 6 h gerührt.

Anschließend wird das Lösungsmittel im Hochvakuum entfernt, der Rückstand mit 25 ml Essigester digeriert, Unlösliches abfiltriert und das Filtrat auf ca. 10 ml eingeengt. Das in wenig Essigester gelöste Produkt wird zu 200 ml Diisopropylether getropft, der Niederschlag abgetrennt, mit Diisopropylether gewaschen und im Trockenschrank mit CaCl getrocknet.
- Ausbeute:: 115 mg (0.20 mmol) 56 % d.Th.
- DC:: Kieselgel (Merck 60), Essigester/Methanol (v/v 2/1)/1 % Eisessig, Detektion mit Kaliumpermanganat R_{f} = 0.83
- ¹H-NMR (D₆-DMSO/TMS):: δ = 1.06-1.50 (m, 12 H, 6 CH₂); 2.08 (t, 2H, CH₂CO, J=6.7 Hz); 2.50 (t, 2H, CH₂COOSu); 2.81 (s, 4H, 2 CH₂COO); 3.31 (m, 6H, 3 CH₂N); 4.12 (m, 1H, CH-NH); 6.98 (s, 4H, CH=); 7.81 (d, 1H, NHCH, J=7.0 Hz); 8.03 ppm (t, br, 1H, NHCH₂).

### Beispiel 2

### 2.1 N-α-Boc-ε-Maleinimido-α-aminohexanoyl-DADOO-Biotin (α-Boc-ε-Mal-Lys-DADOO-Biotin)

Zu einer Lösung aus 1.00 g (3.06 mmol) Verbindung aus Beispiel 1.1 in 50 ml THF werden unter Rühren 422 mg (3.67 mmol) N-Hydroxysuccinimid und 757 mg (3.67 mmol) Dicyclohexylcarbodiimid zugegeben. Nach 1 stündigem Rühren bei 20°C tropft man eine Lösung aus 1.37 g (3.67 mmol) Biotin-DADOO in 50 ml 0.1 M KPO₄-Puffer pH 8.5 zu. Der Ansatz wird nach weitere 2 h bei 20°C gerührt, anschließend das organische Lösungsmittel im Wasserstrahlvakuum entfernt und die wässrige Phase lyophilisiert. Das Rohprodukt wird durch Kieselgelchromatographie, Eluent Essigester/Methanol (v/v 2/1)/1 % Essigsäure, aufgereinigt.
- Ausbeute:: 930 mg (1.36 mmol) 44 % d.Th.
- DC:: Kieselgel (Merck 60), Essigester/Methanol (v/v 2/1)/1 % Eisessig, Detektion mit Biotinspray und Kaliumpermanganat R_{f} = 0.44
- ¹H-NMR (D₆-DMSO/TMS):: δ = 1.10-1.90 (m, 15H, ^{t}Bu u. 6 CH₂); 2.06 (t, 2H, CH₂CO, J=6.7 Hz); 2.60-2.90 (m, 3H, CHS u. CH₂S); 3.00-3.60 (m, 14H, 3 CH₂N u. 4 CH₂O); 3.77 (m, 1H, CHNH-Lys); 4.14 (dd, 1H, CH-NH-Biotin); 4.30 (dd, 1H, CHNH-Biotin); 6.39 (m, 2H, NH-Biotin); 6.76 (d, 1H, NHCOO, J=7.1); 6.99 (s, 2H, CH=); 7.84 ppm (t, br, 2H, 2 NHCO).

### 2.2 ε-Maleinimido-α-aminohexanoyl-DADOO-Biotin (ε-Mal-Lys-DADOO-Biotin) 9

500 mg (0.7 mmol) Verbindung aus Beispiel 2.1 werden bei 0°C mit 10 ml Trifluoressigsäure versetzt und langsam auf 20°C erwärmt. Nach 30 min wird mit 10 ml Essigester verdünnt und weitere 15 min gerührt. Das Lösungsmittel wird im Wasserstrahlvakuum entfernt und der Rückstand aus Dioxan/Wasser (v/v 1/1) lyophilisiert.
- Ausbeute:: ca. 0.7 mmol
- DC:: Kieselgel (Merck 60) Essigester/Methanol (v/v 1/1)/1 % Eisessig, Detektion mit Biotinspray, Kaliumpermanganat und Ninhydrin R_{f} = 0.08

### 2.3 N-α-Maleinimidohexanoyl-ε-Maleinimido-α-aminohexanoyl-DADOO-Biotin (α-MHS-ε-Mal-Lys-DADOO-Biotin, MALOS-Biotin)

0.7 mmol Verbindung aus Beispiel 2.2 werden in 10 ml 0.1 M KPO₄-Puffer pH 7.5 gelöst und mit 260 mg (0.84 mmol) Maleinimidohexansäure-N-hydroxysuccinimid (MHS) in 10 ml THF versetzt. Der pH-Wert wird mit 1 N Natronlauge solange auf pH 7.5 nachgestellt bis keine pH-Wert Änderung mehr zu beobachten ist. Nach 16 h Rühren bei 20°C wird das organische Lösungsmittel im Wasserstrahlvakuum entfernt und der Rückstand lyophilisiert. Die Aufreinigung des Rohprodukts erfolgt durch offene Säulenchromatographie an Kieselgel, Eluent Essigester/Methanol (v/v 1/1)/1 % Essigsäure. Die gepoolten Fraktionen werden im Wasserstrahlvakuum eingeengt, der Rückstand in Dioxan aufgenommen, Ungelöstes abfiltriert und lyophilisiert.
- Ausbeute:: 500 mg (enthält noch Dioxan und Essigsäure)
- DC:: Kieselgel (Merck 60), Essigester/Methanol (v/v 1/1)/1 % Eisessig, Detektion mit Biotinspray und Kaliumpermanganat R_{f} = 0.55
- ¹H-NMR (D₂O/TMS-Na-Salz):: δ = 1.20-1.80 (m, 18 H, 9 CH₂); 2.22 (m, 4H, 2 CH₂CO); 2.71-2.90 (m, 3H, CHS u. CH₂S); 3.38-3.75 (m, 16 H, 4 CH₂N u. 4 CH₂O); 4.20 (m, 1H, CHNH-Lys); 4.44 (dd, 1H, CHNH-Biotin); 4.60 (dd, 1H, CHNH-Biotin); 6.88 ppm (s, 4H, CH=).

### Beispiel 3

### Aktivierung von Proteinen mit erfindungsgemäßen Linkern

### 3.1 Aktivierung von KLH

150 mg ( 5 x 10⁻⁵ mmol/l) Keyhole Limpet Hemocyanin (KLH) werden in 5 ml 0,1 mol/l Kaliumphosphat/0,05 mol/l Natriumchloridpuffer, pH 8,5 gelöst und mit 5 x 10⁻² mmol/l erfindungsgemäßem Linker (Beispiel 1.5) in 2,5 ml Dioxan versetzt. Nach 60 Minuten Rühren bei 20°C wird der pH-Wert mit 0,1 mol/l Natronlauge, pH 8,5 eingestellt und weitere drei Stunden bei 20°C gerührt. Die Aufreinigung erfolgt durch Gelchromatographie an einer ACA 202-Säule 40 x 3 cm, Eluent: 0,1 mol/l Kaliumphosphat/0,05 mol/l Natriumchloridpuffer, pH 7,0).

### 3.2 Aktivierung von POD mit erfindungsgemäßen Linkern

Die Aktivierung erfolgt in einem molaren Überschuß des Linkers (Beispiel 1.5) zu POD von 25 : 1. POD wird mit einer Konzentration von 25 mg/ml in 50 mM Kaliumphosphatpuffer pH 7.5 150 mM NaCl gelöst. Die entsprechende Menge Linker wird in DMSO vorgelöst mit 100 mg/ml. Von dieser Lösung werden 92 µl/ml POD zur POD-Lösung gegeben. Der pH des Ansatzes wird nachkontrolliert und auf 7.0 gestellt. Anschließend wird der Ansatz unter Rühren 1 h/25°C inkubiert. Beendigung der Reaktion erfolgt durch pH-Änderung auf 6.1 und Abkühlung des Ansatzes im Eisbad. Anschließend wird der nicht gekoppelte Linker durch fließende Dialyse gegen 10 mM Kaliumphosphatpuffer pH 6.1, 50 mM NaCl, 1 mM EDTA abgetrennt. Der Einbau der MH-Gruppen wird bestimmt durch Reaktion mit einer definierten Menge an Cystein und Titration der Restmenge an Cystein mit Dithiodipyridin.

### Beispiel 4

### 4.1 Bis(3-phthalimidopropyl)malonsäuredimethylester

1.14 ml (10 mmol) Malonsäuredimethylester und 8.04 g (30 mmol) n-(3-Brompropyl)phtalimid werden unter Stickstoffatmosphäre in 50 ml absolutem Tetrahydrofuran gelöst. Bei 25°C werden 0.24 g (10 mmol) Natriumhydrid zugegeben. Die Mischung wird 8 - 10 h unter Rückfluß erhitzt. Nach dem Abkühlen werden weitere 0.24 g (10 mmol) Natriumhydrid zugegeben und nochmals 20 - 72 h unter Rückfluß erhitzt. Anschließend wird das Lösungsmittel im Wasserstrahlvakuum abdestilliert. Der Rückstand wird in 200 ml Essigester aufgeschlämmt und die Zweiphasensuspension mit 50 ml Wasser gewaschen. Die Wasserphase wird abgetrennt und die verbleibende Suspension filtriert. Der Hauptteil des Produkts wird aus dem Filterkinchen durch Umkristallisieren aus Aceton gewonnen. Das Filtrat wird im Wasserstrahlvakuum zur Trockne eingedampft. Durch Umkristallisieren aus Aceton wird weiteres Produkt gewonnen.
- Ausbeute:: 2.78 mg (5.5 mmol) 55 % d.Th.
- DC:: Kieselgel (Merck 60), Essigester/Hexan (v/v 1/2), Detektion über UV-Absorption R_{f} = 0.55 N-(3-Brompropyl)phthalimid R_{f} = 0.44 (3-Phthalimidopropyl)malonsäuredimethylester R_{f} = 0.32 Bis-(3-phthalimidopropyl)malonsäuredimethylester

### 4.2 5-Amino-2-(3-aminopropyl)pentansäure

1.0 g (2 mmol) Bis-(3-phtalimidopropyl)malonsäuredimethylester aus 4.1 werden in 40 ml Methanol gelöst und mit 10 ml 20 %iger methanolischer KOH (w/v) 15 h bei Raumtemperatur gerührt. Danach wird mit 6 N Salzsäure angesäuert, ausgefallenes Kaliumchlorid wird abgetrennt und das Lösungsmittel im Wasserstrahlvakuum abdestilliert. Der Rückstand wird in 20 ml Mesitylen und 5 ml Eisessig suspendiert und 12 h unter Rückfluß erhitzt. Das Lösungsmittel wird im Hochvakuum abdestilliert, der Rückstand wird 16 h im Exsiccator über Kaliumhydroxid getrocknet. Das Rohprodukt wird in 10 ml Methanol gelöst und mit 5 ml Hydrazinhydrat mehrere Tage unter Rückfluß erhitzt. Das Lösungsmittel und überschüssiges Hydrazinhydrat werden im Hochvakuum abdestilliert, der Rückstand wird mehrmals in Methanol gelöst und wieder im Hochvakuum eingeengt. Danach wird in 1 N Salzsäure dispergiert und vom Ungelösten abfiltriert. Aus der Mutterlauge wird das Produkt durch Kationenaustauschchromatographie (Dowex 50 x 2 - 200) gewonnen (Salzelution mit Wasser, Produktelution mit 1 N - 5 N Ammoniak).
- Ausbeute:: 191 mg (1.1 mol) 55 % d.Th.
- DC:: Kieselgel 60, Wasser/s-Butanol/Eisessig/Essigester (v/v/v/v 1/1/1/1) R_{f} = 0.14 5 Amino-2-(3-aminopropyl)pentansäure
- ¹H-NMR (D₆-DMSO/TMS):: δ = 1.37 (m, 8H, 4CH₂); 2.22 (m, 1H, CH-COO); 2.72 (m, 4H, 2CH₂-N); 8.07 ppm (m, 6H, 2 N⁺H₃).

### 4.3 5-N-Maleinimido-2-(3-N-maleinimidopropyl)pentansäure

420 mg (2 mmol) 5-Amino-2-(3-aminopropyl)pentansäure-Hydrochlorid aus 4.2 in 15 ml gesättigter Natriumhydrogencarbonatlösung werden mit 620 mg (4 mmol) Methoxycarbonylmaleinimid versetzt und unter Eiskühlung gerührt. Nach 3 h werden weitere 350 mg (2.25 mmol) Methoxycarbonylmaleinimid zugegeben und 2 h unter Eiskühlung gerührt. Anschließend stellt man mit 2 M Salzsäure auf pH 4 - 5 und lyophilisiert. Das Lyophilisat wird über präparative HPLC (Säule: Waters Delta-PAK™, C18, 50 X 300 mm, 300 Å, 15 µ, Eluent A: 0.01 % TFA, Eluent B: Acetonitril mit 0.01 TFA, Gradient: 0 % B nach 100 % B, Detektion bei 226 nm) gereinigt.
- Ausbeute:: 216 mg (0.65 mmol) 33 % d.Th.
- DC:: Kieselgel (Merck 60), n-Butanol/Eisessig/Wasser (v/v/v 3/1/1), Detektion mit Kaliumpermanganat-Spray R_{f} = 0.16 5-Amino-2-(3-aminopropyl)pentansäure R_{f} = 0.71 5-N-Maleinimido-2-(3-N-maleinimidopropyl) pentansäure
- Analytische HPLC::
- Säule:: Vydac C18, 4.6 x 250 mm, 5 µ, 300 Å
- Eluent:: A: Millipore-Wasser, 0.01 % TFA
- B:: Actonitril, 0.01 % TFA
- Fluß:: 1 ml/min
- Gradient:: 0 → 50 % B in 45 min
- Detektion:: 226 nm
- Retentionszeiten:: t = 33.3 min 5-N-Maleinimido-2-(3-N-maleinimidopropyl)-pentansäure
- ¹H-NMR(CD₃OD/TMS):: δ = 1.51 (m, 8H, 4CH₂); 2.27 (m, 1H, CH-COO); 3.48 (t, J = 6.7 Hz, 4H, 2 CH₂-N); 6.91 ppm (s, 4H, 2 CH=CH).

### 4.4 5-N-Maleinimido-2-(3-N-maleinimidopropyl)-pentansäure-N-hydroxysuccinimid

200 mg (0.65 mmol) 5-N-Maleinimido-2-(3-N-maleinimidopropyl)-pentansäure werden in 5 ml Tetrahydrofuran gelöst, mit 88.6 mg (0.77 mmol) N-Hydroxysuccinimid und 159 mg (0.77 mmol) Dicyclohexylcabodiimid versetzt und 10 h bei 25°C gerührt. Dann wird die Suspension mit 50 µl Essigsäure versetzt und nach 1 h Rühren über eine Nutsche filtriert. Der abgetrennte Harnstoff wird kurz mit 1 ml Essigester nachgespült, das Filtrat eingeengt, über präp. HPLC (Bedingungen siehe unter 4.3) aufgereinigt und sofort lyophilisiert.
- Ausbeute:: 150 mg (0.35 mmol) 54 % d.Th.
- Analytische HPLC::
- Säule:: Vydac C18, 4.6 x 250 mm, 5 µ, 300 Å
- Eluent:: A: Millipore-Wasser, 0.01 % TFA
- B:: Acetonitril, 0.01 % TFA
- Fluß:: 1 ml/min
- Gradient:: 0 → 50 % B in 45 min
- Detektion:: 226 nm
- Retentionszeiten:: t = 33.3 min 5-N-Maleinamido-2-(3-N-maleinimidoropyl)-pentansäure t = 39.0 Min 5-N-Maleinimido-2-(3-N-maleinimidopropyl)-pentansäure-N-hydroxysuccinimid

## Patentansprüche

1. Verbindung der allgemeinen Formel 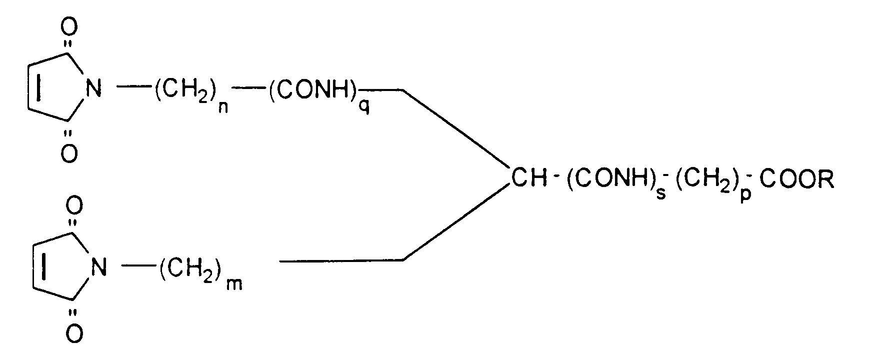 in der R eine esteraktivierende Gruppe, m und n gleich oder verschieden und 0 - 6, q und s 0 oder 1, p = 0, wenn s = 0 ist und p = 2 - 4 wenn s = 1 ist, darstellen.

2. Verbindung nach Anspruch 1, in der
R: Hydroxysuccinimidyl,
n = 5, m = 4, p = 2 und q und s = 1 darstellen.

3. Verbindung nach Anspruch 1, dadurch gekennzeichnet, daß R: Hydroxysuccin imidyl, n und m = 5, p, q und s = O darstellen.

4. Konjugat aus Haptenen und einem Protein, gekoppelt über eine Verbindung gemäß Anspruch 1, wobei die Haptene an deren Thiolgruppen über die Maleinimidofunktionen dieser Verbindung und das Protein über die Funktion der esteraktivierten Gruppe R des Linkers gebunden sind.

5. Konjugat aus zwei ED-Untereinheiten von β-Galactosidase und einem Antikörper, Antikörperfragment oder einem Dextran, bei dem die ED-Untereinheiten an deren Thiolgruppen über zwei Maleinimidofunktionen der Verbindung nach Anspruch 1 und der Antikörper, das Antikörperfragment oder das Dextran über die Funktion der esteraktivierten Gruppe R gebunden sind.

6. Verfahren zur Herstellung von Verbindungen der allgemeinen Formel, 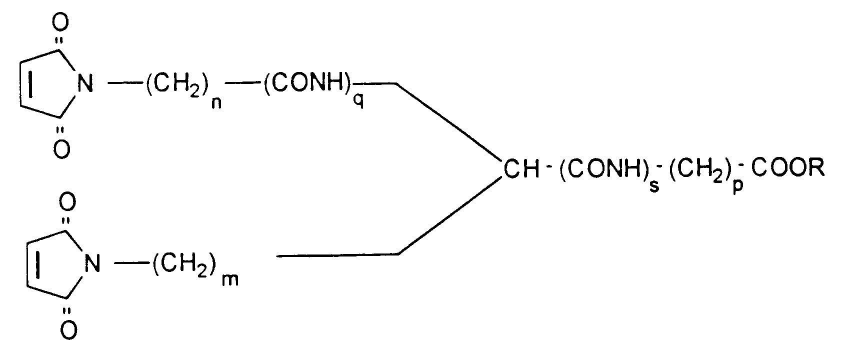 in der R eine esteraktivierende Gruppe,
m und n gleich oder verschieden und 0 - 6
p = 0, q = 0 und s = 0 darstellen durch
a) zweifache Malonestersynthese mit N-Phtalimidoalkylhalogenid an Malonsäurediester zu einem Dialkylmalonsäurediester
b) Esterspaltung unter sauren Bedingungen und Decarboxylierung
c) Saure Hydrolyse zur Freisetzung der Aminogruppen aus den N-Phtalimidoalkylgruppen
d) Einführung von zwei Maleinimidylresten durch Behandlung mit Alkyloxycarbonylmaleinimid
e) Aktivierung der Carboxyfunktion zu einem aktivierten Ester.

7. Verfahren zur Herstellung von Verbindungen der allgemeinen Formel, 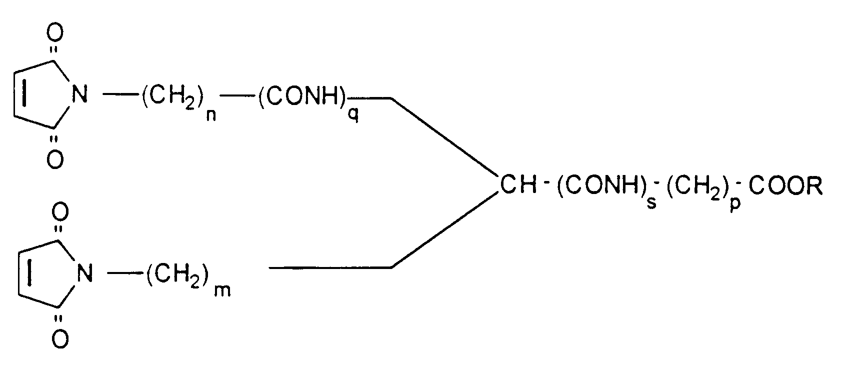 in der R eine esteraktivierende Gruppe
m und n gleich oder verschieden und 0 - 6 p = 0, q = 0 und s = 1 darstellen, durch
a) zweifache Malonestersynthese mit N-Phtalimidoalkylhalogenid an Malonsäurediester zu einem Dialkylmalonsäurediester
b) Esterspaltung unter sauren Bedingungen und Decarboxylierung
c) Saure Hydrolyse zur Freisetzung der Aminogruppen aus den N-Phtalimidoalkylgruppen
d) Einführung von zwei Maleinimidylresten durch Behandlung mit Alkyloxycarbonylmaleinimid
e) Aktivierung der Carboxyfunktion zu einem aktivierten Ester.
f) Umsetzung des aktivierten Esters mit einer ω-Aminocarbonsäure und
g) Aktivierung der Carboxyfunktion zu einem aktivierten Ester.

8. Verfahren zur Herstellung von Verbindungen der allgemeinen Formel 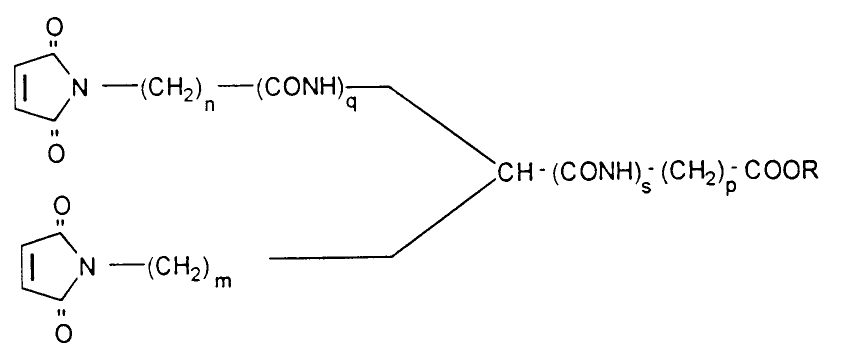 in der R eine esteraktivierende Gruppe,
m und n gleich oder verschieden und 0 - 6 p = 0, q = 1 und s = 1 darstellen durch
a) Umsetzung einer Diaminosäure, in der eine der Aminofunktionen durch eine säurelabile Schutzgruppe geschützt ist, mit Alkoxycarbonylmaleinimid
b) Umsetzung der freien Carboxygruppe mit einer Aminocarbonsäure
c) Abspaltung der Schutzgruppe unter sauren Bedingungen
d) Umsetzung der erhaltenen Verbindung mit einer carboxyaktivierten Maleinamidoalkansäure
e) und Aktivierung der freien Carboxyfunktion zu einem aktivierten Ester.

9. Konjugat aus zwei Haptenen und einer ED-Untereinheit von β-Galactosidase, bei dem die Haptene an deren SH-Gruppen über zwei Maleinimidofunktionen der Verbindung nach Anspruch 1 und die ED-Untereinheit über die Funktion der esteraktivierten Gruppe R gebunden sind.

## Claims

1. Compound of the general formula 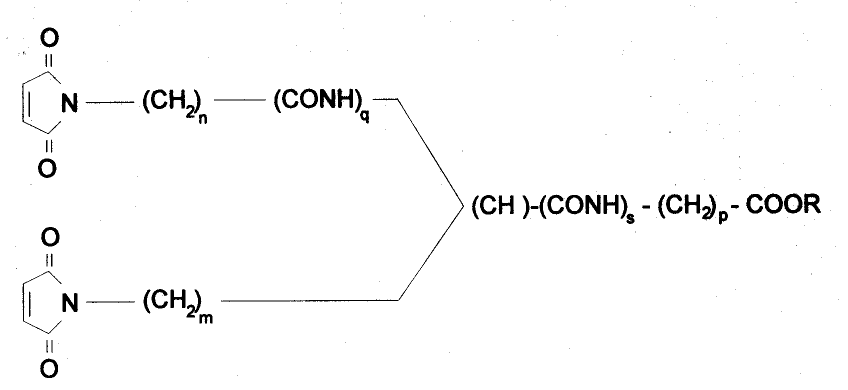 wherein R is an ester-activating group, m and n are identical or different and 0 - 6, q and s are 0 or 1, p = 0 if s = 0, and p = 2 - 4 if s = 1.

2. Compound according to claim 1, wherein
R: hydroxysuccinimidyl,
n = 5, m = 4, p = 2, and q and s = 1.

3. Compound according to claim 1, characterized in that
R: hydroxysuccinimidyl, n and m = 5, p and q and s = 0.

4. Conjugate of haptens and one protein, coupled via a compound according to claim 1, wherein the haptens are linked to the thiol groups thereof via the maleinimido group of this compound, and the protein is linked via the functional group of teh ester-activating group R of the linker.

5. Conjugate consisting of two ED-β-galactosidase subunits and one antibody, antibody fragment or a dextran wherein the ED subunits, at their thiol groups, are linked via two maleinimido groups of the compound according to claim 1 and the antibody, the antibody fragment or the dextran is linked via the functional group of the ester-activating group R.

6. Method of preparing compounds of the general formula 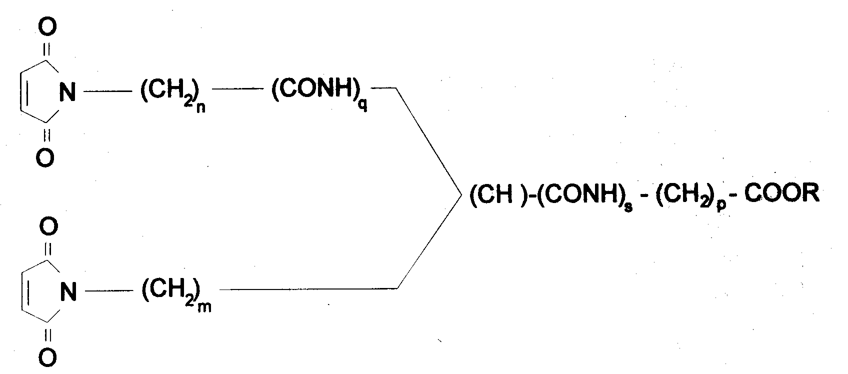 wherein R is an ester-activating group, m and n are identical or different and 0 - 6, p = 0, q = 0, and s = 0, by means of
a) two-fold malonic ester synthesis with N-phthalimidoalkylhalogenide and malonic acid diester to form a dialkylmalonic acid diester
b) ester cleavage under acidic conditions and decarboxylation
c) hydrolysis in an acidic milieu to release amino groups from the N-phthalimidoalkyl groups
d) incorporation of two maleinimido residues by treatment with alkyloxycarbonylmaleinimide
e) activating the carboxyl groups to form an activated ester.

7. Method of preparing compounds of the general formula 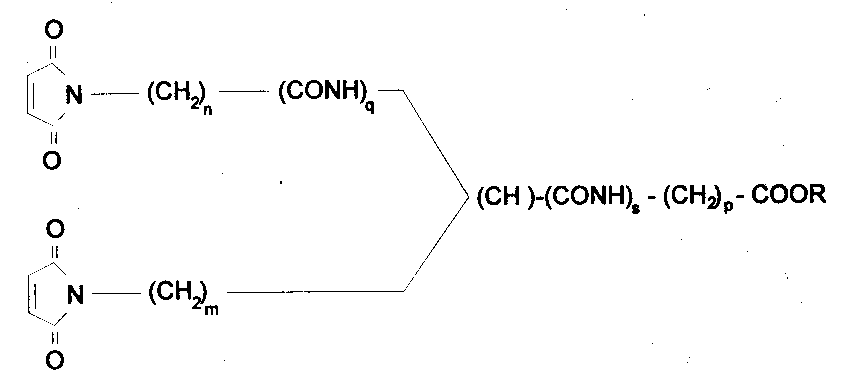 wherein R is an ester-activating group, m and n are identical or different and 0 - 6, p = 0, q = 0, and s = 1, by means of
a) two-fold malonic ester synthesis with N-phthalimidoalkylhalogenide and malonic acid diester to form a dialkylmalonic acid diester
b) ester cleavage under acidic conditions and decarboxylation
c) hydrolysis in an acidic milieu to release amino groups from the N-phthalimidoalkyl groups
d) incorporation of two maleinimido residues by treatment with alkoxycarbonylmaleinimide
e) activating the carboxyl groups to form an activated ester
f) reaction of the activated ester with an ω-amino carboxylic acid and
g) activating the carboxyl group to form an activated ester.

8. Method of preparing compounds of the general formula 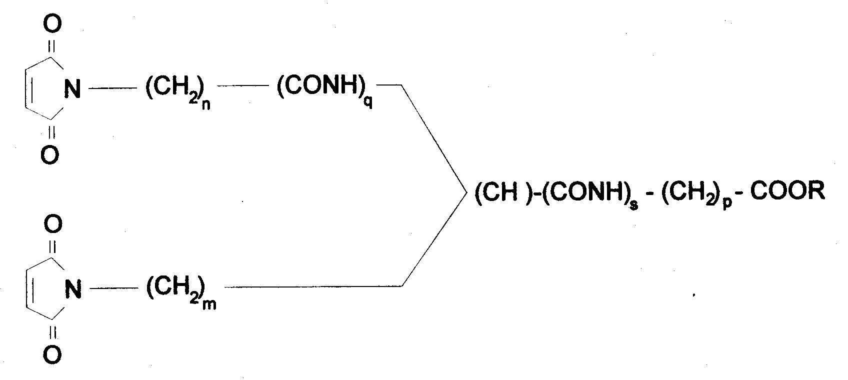 wherein R is an ester-activating group, m and n are identical or different and 0 - 6, p = 0, q = 1, and s = 1, by means of
a) reacting a diamino acid with alkoxycarbonylmaleinimide, where in one functional amino group is protected by an acid-labile protective group
b) reacting the free carboxyl group with an amino carboxylic acid
c) cleaving off the protective group under acidic conditions
d) reacting the resulting compound with a carboxy-activated alkyl carboxylic acid
e) and activating the free carboxyl group to form an activated ester.

9. Conjugate consisting of two haptens and one β-glactosidase ED subunit wherein the haptens, at their SH groups, are linked via two maleinimido groups of the compound according to claim 1 and the ED subunit is linked via the functional group of the ester-activating group R.

## Revendications

1. Composé de formule générale 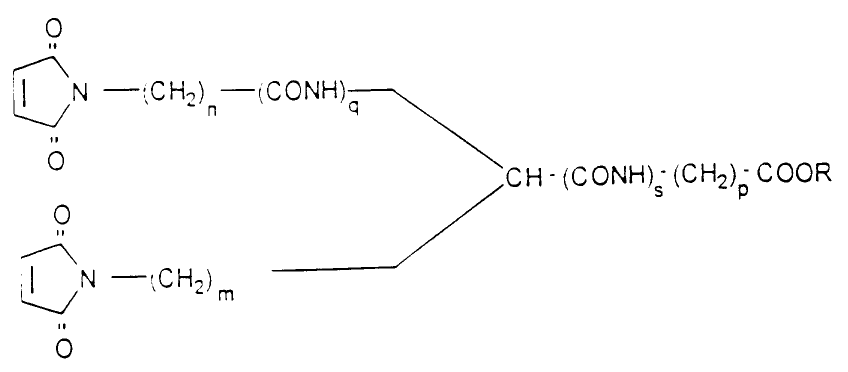 dans laquelle R est un groupe activateur d'ester, m et n sont identiques ou différents et valent 0-6, q et s valent 0 ou 1, p=0 lorsque s=0 et p=2-4 lorsque s=1.

2. Composé selon la revendication 1, dans lequel
R : hydroxysuccinimidyle,
n=5, m=4, p=2 et q et s = 1.

3. Composé selon la revendication 1, caractérisé par le fait que R : hydroxysuccinimidyle, n et m = 5, p, q et s = 0.

4. Conjugué d'haptènes et d'une protéine couplés par l'intermédiaire d'un composé selon la revendication 1, dans lequel les haptènes sont liés sur leurs groupes thiols par l'intermédiaire des fonctions maléinimido de ce composé et la protéine par l'intermédiaire de la fonction du groupe R d'ester activé du linker.

5. Conjugué de deux sous-unités DE de β-galactosidase et d'un anticorps, d'un fragment d'anticorps ou d'un dextrane, dans lequel les sous-unités ED sont liées sur leurs groupes thiols par l'intermédiaire de deux fonctions maléinimido du composé selon la revendication 1 et l'anticorps le fragment d'anticorps ou le dextrane sont liés par l'intermédiaire de la fonction du groupe R d'ester activé.

6. Procédé de préparation de composés de formule générale 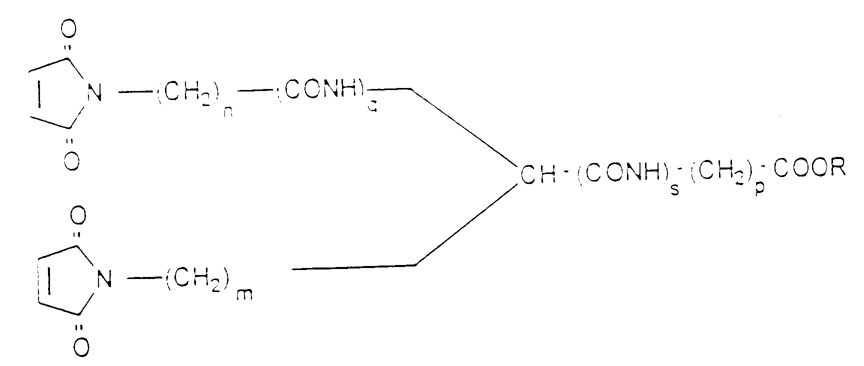 dans laquelle R est un groupe activateur d'ester, m et n sont identiques ou différents et valent 0-6, p=0 q=0 et s=0, comprenant les étapes consistant à effectuer
a) deux synthèses d'ester malonique avec un halogénure de N-phtalimidoalkyle sur un diester malonique pour former un diester dialkylmalonique,
b) un clivage d'ester dans des conditions acides et une décarboxylation,
c) une hydrolyse acide pour libérer les groupes amino à partir des groupes N-phtalimidoalkyle,
d) l'introduction de deux restes maléinimidyle par traitement avec un alkyloxycarbonylmaléinimide,
e) l'activation de la fonction carboxyle pour former un ester activé.

7. Procédé de préparation de composés de formule générale 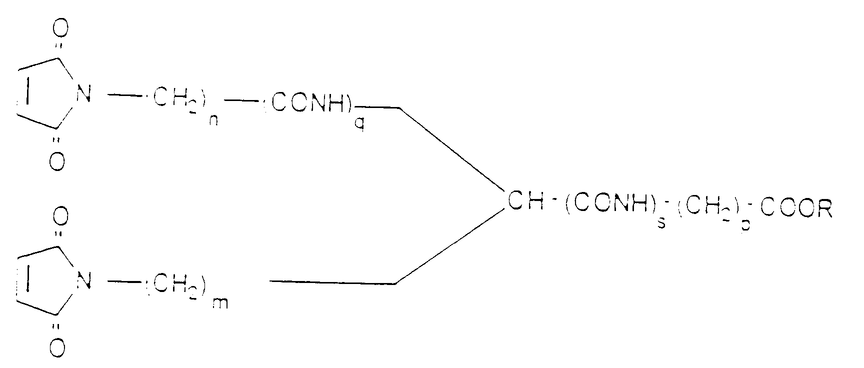 dans laquelle R est un groupe activateur d'ester, m et n sont identiques ou différents et valent 0-6, p=0, q=0 et s=1, comprenant les étapes consistant à effectuer
a) deux synthèses d'ester malonique avec un halogénure de N-phtalimidoalkyle sur un diester malonique pour former un diester dialkylmalonique,
b) un clivage d'ester dans des conditions acides et une décarboxylation,
c) une hydrolyse acide pour libérer les groupes amino à partir des groupes N-phtalimidoalkyle,
d) l'introduction de deux restes maléinimidyle par traitement avec un alkyloxycarbonylmaléinimide.
e) l'activation de la fonction carboxyle pour former un ester activé,
f) la réaction de l'ester activé avec un acide ω-aminocarboxylique, et
g) l'activation de la fonction carboxyle pour former un ester activé.

8. Procédé de préparation de composés de formule générale 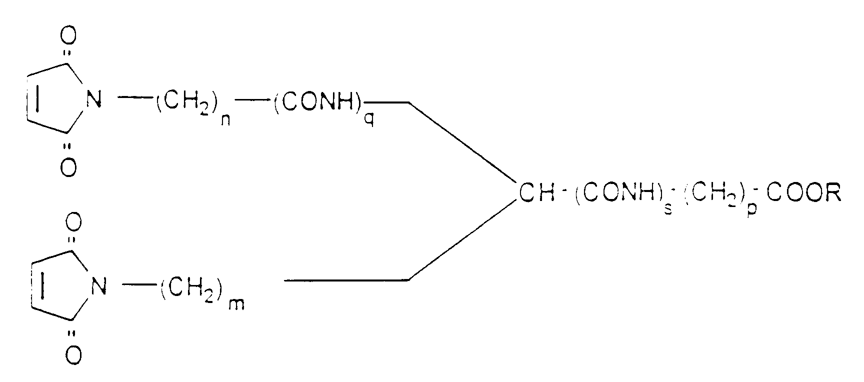 dans laquelle R est un groupe activateur d'ester, m et n sont identiques ou différents et valent 0-6, p=0, q=1 et s=1, comprenant les étapes consistant
a) à faire réagir un diaminoacide, dans lequel une des fonctions amine est protégée par un groupe protecteur déplaçable par acide, avec un alcoxycarbonylmaléinimide,
b) à faire réagir le groupe carboxyle libre avec un acide aminocarboxylique,
c) à séparer le groupe protecteur dans des conditions acides,
d) à faire réagir le composé obtenu avec un acide d'un maléinamidoalcalcane à fonction carboxyle activée, et
e) à activer la fonction carboxyle libre pour former un ester activé.

9. Conjugué de deux haptènes et d'une sous-unité DE de β-galactosidase, dans lequel les haptènes sont liés, sur leurs groupes SH, par l'intermédiaire de deux fonctions maléinimido du composé selon la revendication 1, et la sous-unité DE est liée par l'intermédiaire de la fonction du groupe d'ester activé.
